# EUROPEAN PATENT APPLICATION

(11) **EP 4 299 714 A1**
(43) Date of publication of application: **03.01.2024**
(21) Application number: 22759164.1
(22) Date of filing: 18.01.2022
(51) Int. Cl.: C12M 1/34, C12Q 1/06, C12Q 1/6837, C12Q 1/6844, C12Q 1/6888

(54) **MEASUREMENT METHOD AND MEASUREMENT SYSTEM**

(30) Priority: 26.02.2021 JP 2021030936
(71) Applicant: Yokogawa Electric Corporation, Tokyo 180-8750 (JP)
(72) Inventor: TAGUCHI Tomoyuki, Musashino-shi, Tokyo 180-8750 (JP); MIYAUCHI Yuki, Musashino-shi, Tokyo 180-8750 (JP); KATAYAMA Hiroyuki, Tokyo 113-8654 (JP)
(74) Representative: Winter, Brandl - Partnerschaft mbB
(86) International application number: PCT/JP2022/001643
(87) International publication number: WO 2022/181121

(57) **Abstract**

A measurement method for quantitatively measuring amounts of at least two varieties of microorganisms in a test sample. The measurement method includes: a step of producing a first sample by performing step dilution and division of the test sample; a step of producing a second sample by amplifying nucleic acids of the microorganisms in the first sample; and a step of performing hybridization of the second sample in a probe array 20 including spots 30 to which probes 40 having complementary sequences to target nucleic acids specific to each of the varieties of the microorganisms are fixed; and a step of identifying the varieties of the microorganisms based on light emission from the spots 30 where the hybridization is performed and quantitatively measuring amounts of the microorganisms by a most probable number method.

## Description

### CROSS-REFERENCE TO RELATED APPLICATION

The present application claims priority to and the benefit of Japanese Patent Application No. 2021-30936 filed February 26, 2021, the entire contents of which are incorporated herein by reference.

### TECHNICAL FIELD

The present disclosure relates to a measurement method and a measurement system for measuring at least two varieties of microorganisms.

### BACKGROUND

Conventionally, methods for simultaneously detecting the presence or absence of two or more genes are known (see, for example, Patent Literature (PTL) 1).

### CITATION LIST

### Patent Literature

PTL 1: JP 2015-42152 A

### SUMMARY

### (Technical Problem)

There is a demand for improved convenience in microorganism measurement.

It would be helpful to provide a measurement method and a measurement system capable of improving convenience in microorganism measurement.

### (Solution to Problem)

A measurement method according to at least one embodiment is for quantitatively measuring amounts of at least two varieties of microorganisms present in a test sample. The measurement method comprises: a step of producing a first sample by performing step dilution and division of the test sample; a step of producing a second sample by amplifying nucleic acids of the microorganisms in the first sample; and a step of performing hybridization of the second sample in a probe array including spots to which probes corresponding to each of the varieties of the microorganisms are fixed; and a step of identifying the varieties of the microorganisms based on light emission from the spots where the hybridization is performed and quantitatively measuring amounts of the microorganisms by a most probable number method. In this way, multiple varieties of microorganisms may be easily detected. As a result, convenience in microorganism measurement is improved.

According to an embodiment, the measurement method further comprises a step of designing a primer to be added to the first sample based on a variety of probe fixed in the probe array. In this way, target microorganisms may be easily changed. As a result, convenience in microorganism measurement is improved.

A measurement system according to at least one embodiment is for quantitatively measuring amounts of at least two varieties of microorganisms present in a test sample. The measurement system comprises a pretreatment device and a measurement device. The pretreatment device is operable to produce a first sample by step dilution and division of the test sample, produce a second sample by amplification of nucleic acids of the microorganisms in the first sample, and perform hybridization of the second sample in a probe array including spots to which probes having complementary sequences for target nucleic acids specific to each of the varieties of the microorganisms are fixed. The measurement device is operable to identify the varieties of the microorganisms based on light emission from the spots where the hybridization is performed and to quantitatively measure amounts of the microorganisms by a most probable number method. In this way, multiple varieties of microorganisms may be easily detected. As a result, convenience in microorganism measurement is improved.

### (Advantageous Effect)

The measurement method and the measurement system according to the present disclosure improve convenience in microorganism measurement.

### BRIEF DESCRIPTION OF THE DRAWINGS

In the accompanying drawings:
FIG. 1 is a flowchart illustrating procedures of pre-processing of a multiplex polymerase chain reaction (PCR) amplification method according to a comparative example;
FIG. 2 is a flowchart illustrating procedures of quantitative measurement of target nucleic acids in a test sample according to a comparative example;
FIG. 3 is a flowchart illustrating procedures of a measurement process for quantifying target nucleic acids in a test sample by a quantitative multiplex nucleic acid amplification reaction according to a comparative example;
FIG. 4 is a flowchart illustrating procedures of quantitative measurement of target nucleic acids in a test sample by performing a digital PCR amplification reaction according to a comparative example;
FIG. 5 is a flowchart illustrating example procedures of pre-processing for quantitative measurement of microorganisms present in a test sample in a measurement method according to an embodiment;
FIG. 6 is a flowchart illustrating example procedures of processing for quantitative measurement of microorganisms present in a test sample in a measurement method according to an embodiment;
FIG. 7 is a schematic diagram illustrating an example configuration of a measurement device included in a measurement system according to an embodiment;
FIG. 8 is a schematic diagram illustrating an example configuration of a probe array in which a target nucleic acid is hybridized to a probe;
FIG. 9 is a schematic diagram illustrating an example configuration in which light-emitting spots and non-light-emitting spots are discriminated in a probe array; and
FIG. 10 is a schematic diagram illustrating an example configuration of a pretreatment device included in a measurement system according to an embodiment.

### DETAILED DESCRIPTION

Embodiments of the present disclosure are described below with reference to the drawings. A measurement method according to an embodiment of the present disclosure is a method for quantitatively measuring amounts of at least two varieties of microorganisms present in a test sample.

### (Comparative examples)

First, comparative examples are described to compare with the measurement method according to the present embodiment.

### <Colony count method and most probable number (MPN) method>

One method that may be employed to quantitatively measure target microorganisms in a test sample is a colony count method. In the colony count method, colonies formed by target microorganisms are visually counted. An advantage of the colony count method is that visual identification and counting of microorganism colonies is easy to perform. However, target microorganisms that are detectable by the colony count method are limited to those that are able to form colonies. Further, when the number of microorganisms in the test sample is small, visual measurement results vary greatly.

Another method is a most probable number method. The most probable number method is also referred to as the MPN method. The MPN method is used when the number of microorganisms in a test sample is small. The MPN method is a method for estimating the amount of microorganisms contained in a test sample by comparing the culture results of a test sample divided into multiple parts with a most probable number table calculated according to statistical probability theory.

In order to measure microorganisms in the colony count and MPN methods, visualizing microorganisms using microorganism growth potential is necessary. Therefore, measuring a target microorganism by the colony count and MPN methods is difficult when a culture method for the target microorganism has not been established or when the target microorganism is a viable but non-culturable (VBNC) microorganism.

Further, microorganisms in a test sample collected from the environment may be damaged due to environmental stresses and other factors. Microorganism damage may result in loss or suppression of the original growth potential of the microorganism. When measuring an amount of damaged microorganisms by a method that requires the culturing of microorganisms, as described above, there is a risk that the number of microorganisms may be underestimated.

Further, when culturing multiple target microorganisms for measurement, dividing the test sample by the number of target microorganism varieties is necessary in order to use a suitable growth medium for each target microorganism. As a result, measurement workload increases as the number of target microorganisms increases. Further, when a test sample amount is small, dividing the test sample becomes difficult.

### <Polymerase chain reaction (PCR) method>

A method may be employed of quantitatively measuring the number of microorganisms in a test sample by a molecular biology technique. Specifically, the number of microorganisms may be counted by quantitatively measuring biomolecules such as nucleic acids contained in the target microorganisms using a molecular biology technique. For example, MPN-PCR, quantitative PCR (qPCR), digital PCR, microfluidic qPCR (MF qPCR), and the like may be employed as molecular biology techniques. These methods are based on the application of nucleic acid amplification methods, collectively known as PCR methods. PCR methods require oligonucleotides (short-chain nucleic acids) called primer pairs to initiate the reaction necessary to amplify the target nucleic acid, a heat-resistant enzyme called polymerase to extend a template nucleic acid chain, or deoxynucleotide triphosphate (dNTP) bases, which are a material for nucleic acid chain extension. In a PCR method, pairs of primers with specificity for each target nucleic acid are required to amplify the target nucleic acids. Similar to the MPN method described above, when detecting multiple varieties of target microorganisms, PCR reactions must be performed for each target microorganism. As a result, the number of operations and measurement costs increase as the number of varieties of nucleic acids to be amplified increases.

Further, multiplex PCR may be employed as a technique to amplify multiple target nucleic acids in a single reaction by designing primers without mutual interference. The number of microorganisms may be counted by measuring nucleic acids amplified by multiplex PCR amplification.

### < <Procedures for building a primer set> >

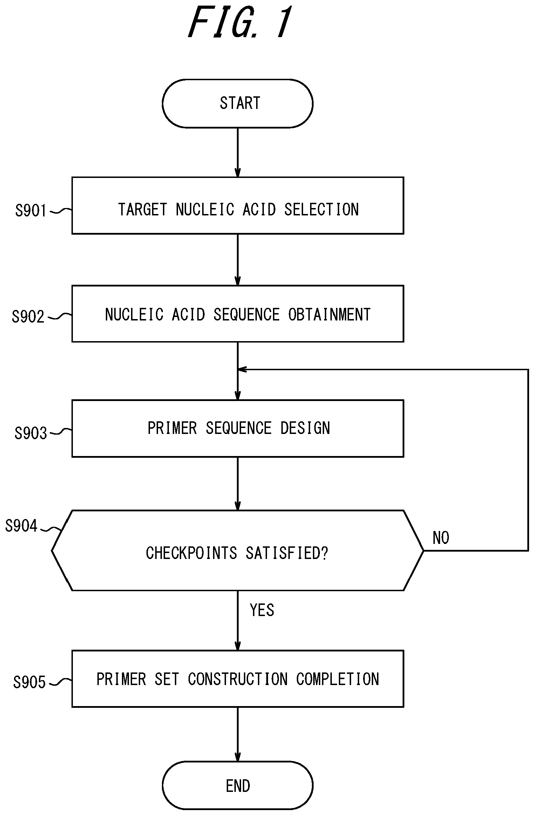

FIG. 1 illustrates procedures for constructing a primer set as pre-processing required to perform multiplex PCR amplification for simultaneous detection of multiple nucleic acids based on the MPN-PCR method. First, a nucleic acid specific to a target microorganism is selected as a target nucleic acid from among genes or other nucleic acids of the target microorganism (step S901). Next, a nucleic acid sequence of the target nucleic acid selected in step S901 is obtained (step S902). The nucleic acid sequence of the target nucleic acid may be obtained from a nucleic acid sequence contained in publicly available nucleic acid sequence databases or the like, or may be obtained by nucleic acid sequencing or other techniques.

Next, primer sequences necessary to amplify the target nucleic acid are designed based on the target nucleic acid sequence obtained in step S902 (step S903). The primer sequences are designed so that Tm values are equivalent between the forward and reverse primers. The Tm value, also referred to as the melting temperature, represents the temperature at which the primer forms a double strand with the target sequence 50 % of the time. Further, a PCR amplification reaction is performed to confirm that the target nucleic acid can be efficiently amplified by the designed primers. The PCR amplification reaction is performed in a buffer solution containing the template nucleic acid, bases, and elongase required for the reaction, as well as salts required for the enzymatic reaction, all based on publicly available information. Further, the PCR amplification reaction is controlled by a temperature cycle appropriate for the enzyme used, consisting of three steps: annealing, extension, and thermal denaturation. The temperature cycle may consist of two steps where extension and thermal denaturation are performed at the same temperature.

Following the PCR amplification reaction, whether checkpoints have been satisfied is determined by gel electrophoresis or the like, to confirm that PCR amplification products have been produced (step S904). Checkpoints include amount of amplification, amplification length, the presence or absence of amplification of non-specified products, the presence or absence of primer dimer formation, and the like. When checkpoints do not satisfy requirements, an improvement or redesign of the primers is performed. Then confirmation is required that the improved or redesigned primers are capable of amplifying the target nucleic acid. Primers designed to satisfy the checkpoint conditions are determined as individual stand-alone primers.

Further, when multiple varieties of nucleic acids are to be detected simultaneously, individual stand-alone primers must be constructed for each variety of nucleic acid. After constructing individual stand-alone primers for all target nucleic acids, PCR amplification is performed on a primer set that is a mix of the individual stand-alone primers constructed for each variety of nucleic acid. Then, to confirm that PCR amplification with the primer set has produced PCR amplification products, whether or not checkpoints have been satisfied is determined. The checkpoints for PCR amplification with the primer set may be the same as those for PCR amplification with individual stand-alone primers.

When PCR amplification with the primer set does not satisfy the checkpoint conditions, due to the production of a non-specified product for example, (step S904: NO), processing returns to step S903 and the individual stand-alone primer is redesigned. When the checkpoint conditions are satisfied (step S904: YES), the construction of a primer set in which non-specified products do not occur is completed (step S905). On determining in step S904 that designing a primer that meets the checkpoint conditions in step S904 is impossible due to a large number of microorganism varieties, processing may return to step S901.

### < < Quantitative measurement of target nucleic acids>>

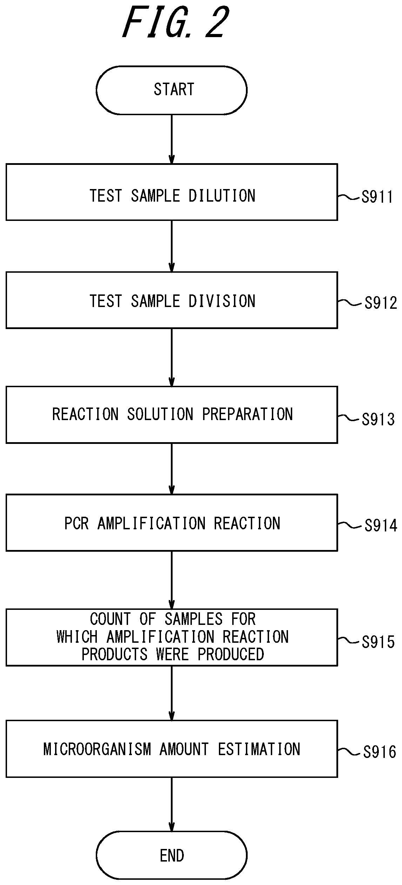

Next, using the primer set constructed in the pre-processing, a nucleic acid amplification reaction is performed to quantitatively measure the target nucleic acids in the test sample by the MPN-PCR method, as illustrated in FIG. 2.

First, the test sample is appropriately diluted to an extent that PCR amplification can be confirmed, based on a most probable number table using the test sample (step S911). In this example, the test sample is diluted in three steps. In other words, the test sample is diluted at three different concentrations. Next, each sample diluted to the same concentration is divided based on the most probable number table (step S912). In this example, the diluted sample is divided into three parts.

Next, a reaction solution is prepared by adding to the divided samples the thermostable enzyme, nucleotides, and primer set constructed in the pre-processing, which are required for the PCR amplification reaction (step S913). Depending on the variety of the target microorganism, an additional step of extracting the target nucleic acid as a template may be added. The step of extracting the target nucleic acid as a template may be added before step S911 or step S912.

Next, a PCR amplification reaction is performed on the sample to which the reaction solution has been added under conditions suitable for the primers (step S914). The PCR amplification reaction is controlled by an appropriate temperature cycle. Next, the number of samples for which PCR amplification products were produced is counted for the samples for which amplification reactions were performed (step S915). Confirmation may be performed by gel electrophoresis or other means. Next, the number of samples for which the production of PCR amplification products is confirmed is checked against the most probable number table, and the amount of microorganisms in the test sample is estimated based on matching results (step S916).

As described above, the amount of the microorganisms in the test sample is estimated based on the MPN-PCR method. Alternatively, the amount of the microorganisms in the the test sample is estimated based on the qPCR method. Even when multiple nucleic acids are quantitatively measured simultaneously based on the qPCR method, a primer set is required, as in the MPN-PCR method. However, the primer set used in the qPCR method must be constructed in such a way that it strictly does not produce non-specified reactions, more so than the primer set used in the MPN-PCR method. Further, in quantitative nucleic acid amplification reactions using an intercalator (a fluorescent reagent that nonspecifically stains double-stranded nucleic acids) or a fluorescent nucleic acid probe (also referred to as a TaqMan probe), a small amount of non-specified amplification product or primer dimers may be produced. This production induces an increase in non-specified fluorescence, which has a significant impact on the estimation of target nucleic acids. As a result, measurement uncertainty increases.

### <<Measurement of target nucleic acids by multiplex nucleic acid amplification reaction> >

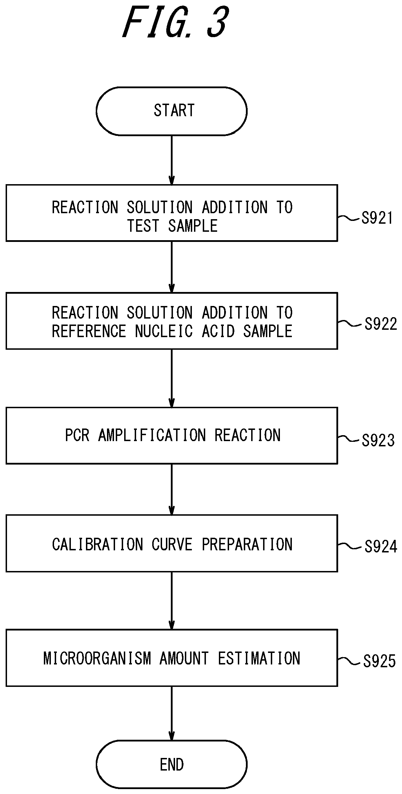

Next, procedures of a measurement process for quantifying the target nucleic acids in the test sample by a quantitative multiplex nucleic acid amplification reaction are illustrated in FIG. 3.

In the quantitative multiplex amplification reaction, the reaction solution required for the amplification reaction is added to the test sample (step S921). Before the procedure of step S921, an additional step of extracting the target nucleic acids from the test sample as in the MPN-PCR method may be added. A reaction solution similar to the reaction solution used in step S913 of FIG. 2 is added as the reaction solution. Further, in order to perform the quantitative amplification reaction, an additional reagent is needed to monitor the progress of the reaction. As a reagent for monitoring, a reagent used in a known method such as an intercalator, fluorescent nucleic acid probe, or the like may be applied.

In parallel with the process of adding the reaction solution and reagent to the test sample in the procedure of step S921, a reaction solution is added to a reference nucleic acid sample for the preparation of a calibration curve, which is necessary for estimating the target nucleic acid concentration (step S922). The reference nucleic acid sample is processed in the same manner as the test sample, but must be reacted in a different reaction system than the test sample.

The amplification reaction is performed on the reaction solution prepared in steps S921 and S922 in the same manner as in step S914 of FIG. 2 (step S923). When performing the quantitative amplification reaction, an instrument capable of measuring fluorescence intensity at the end of each temperature cycle is used. Next, a calibration curve for calculating nucleic acid concentration is prepared based on the measurement results of the reference nucleic acids obtained from the reaction performed in step S923 (step S924). Further, the Cq value is calculated based on the measurement results of the test sample. The Cq value represents the number of cycles when a certain amount (threshold) of PCR amplified product is reached. Next, the amount of microorganisms present in the test sample is calculated based on the calibration curve created in step S924 and the calculated Cq value (step S925).

### <<Measurement procedures in digital PCR method>>

In the pre-processing for simultaneous detection of multiple nucleic acids based on a digital PCR method, constructing individual stand-alone primers by performing the steps S901 through S905 illustrated in FIG. 1 is necessary, as in the MPN-PCR method. In digital PCR that performs simultaneous multiple amplification, as in the quantitative nucleic acid amplification method, the production of non-specified products greatly affects the measurement results, so constructing a primer set that does not produce non-specified products is necessary.

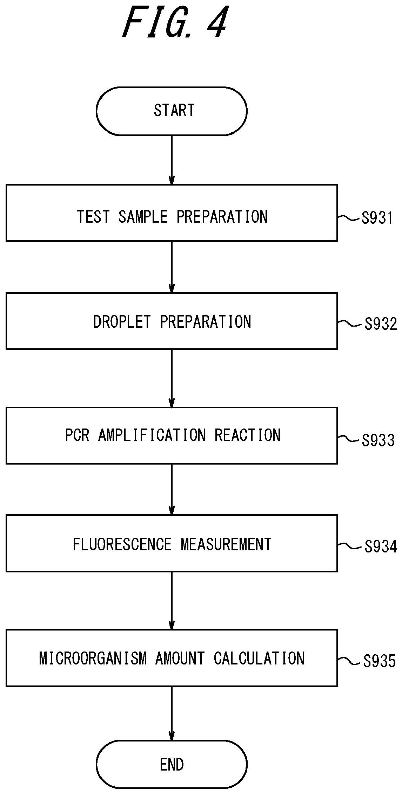

FIG. 4 illustrates procedures of the quantitative measurement of target nucleic acids in a test sample by performing a digital PCR amplification reaction. First, the test sample is prepared (step S931). The preparation of the test sample is performed in procedures similar to steps S911 through S913 in FIG. 2.

In the digital PCR method, droplets are then prepared using the reaction solution prepared in the procedure of step S913 (step S932). Droplets are minute droplets formed by dispersing the reaction solution, which is an aqueous solution, into oil or the like that does not mix with the aqueous solution. Droplets are prepared to contain only one molecule of nucleic acid theoretically extracted from the test sample.

Next, an amplification reaction is performed on the droplets (step S933). The amplification reaction is performed in a procedure similar to that of step S914 in FIG. 2. In digital PCR, as in the quantitative nucleic acid amplification method, a fluorescent reagent such as an intercalator is added to confirm the progress of the reaction.

Next, the amount of fluorescence of each droplet is measured after the amplification reaction (step S934). The amount of microorganisms contained in the test sample is then calculated based on the results of the fluorescence measurement (step S935). The amount of microorganisms in the test sample is calculated based on the quantity of droplets with increased fluorescence being equal to the quantity of target nucleic acids present in the test sample. This is referred to as digital PCR because the quantity of droplets is countable digitally.

### <Summary of comparative examples>

As described above, various methods may be considered as comparative examples of methods for quantitatively measuring the amount of microorganisms in a test sample. The various methods have the following problems.

In the colony counting method, the target microorganisms to be measured are limited to those that can be cultured. The method is not applicable to microorganisms that cannot be cultured because such microorganisms are not identifiable as colonies. When the target microorganisms are present in a small amount in the test sample, the accuracy of the measurement is low. Not all samples can be subjected to the colony counting method. Therefore, the measurement results depend on sampling probability. Unspecified target microorganisms are measured. In other words, the identification of bacterial species is not performed at the same time. Growth media that may be used to specifically culture only certain microorganism species are limited. Therefore, using a growth medium with a wide culture spectrum is usually necessary. A culture-based method requires a long measurement time. For example, in the case of slow-growing target microorganisms, the time required for measurement may be as long as two weeks. Culture rates are highly dependent on culture conditions such as temperature, growth medium composition, oxygen supply, and the like. Therefore, culturing under uniform conditions is difficult. Culture conditions need to be changed for each target microorganism.

In the MPN method, the microorganisms to be measured are limited to those that can be cultured. Unspecified target microorganisms are measured. A culture-based method requires a long measurement time. For example, in the case of slow-growing target microorganisms, the time required for measurement may be as long as two weeks. The MPN method is based on a culture method similar to the colony counting method described above. Therefore, the problems with the MPN method are similar to those of the colony counting method. Step dilution is required. Thus, the number of cultures is increased. This results in increased labor and cost.

In the MPN-PCR method (multi-MPN PCR method), step dilution is required. Thus, the number of reactions is high. This results in increased labor and cost. Three-step dilution and three divisions are the minimum reduction required. Therefore, at least 9 reactions must be performed. The number of target microorganisms is limited to, for example, 5 varieties. To confirm the PCR amplification reaction by electrophoresis, the amplification length must be adjusted based on gel concentration. On the other hand, PCR amplification efficiency is affected when amplification lengths differ significantly. Therefore, amplification lengths are preferably approximated. These limitations limit the number of target microorganisms that are simultaneously measurable. Time and effort are required to add target microorganisms after the primer set has been constructed. Individual stand-alone primers need to be constructed for each target microorganism to be added. An individual stand-alone primer constructed for the target microorganism to be newly added to the primer set that has already been constructed is then added. Confirmation of the amplification reaction is performed for the primer set to which the new individual stand-alone primer has been added. When there is a failure in this check, starting over from the construction of the individual stand-alone primer becomes necessary. As the number of target microorganisms increases, the probability of production of a non-specified product increases. Thus, a great deal of time and effort are required to build the primer set. Further, a situation may arise where a suitable primer set cannot be constructed. In this case, starting over from the first procedure illustrated in FIG. 1 may be necessary.

In the qPCR method (a real-time PCR method), the number of target microorganisms is limited to about six varieties, for example, by a limit based on fluorescence wavelength. Amplification must be confirmed at each reaction cycle. Primers must be designed so that a non-specified PCR amplification product does not occur. The reference sample for calibration must be submitted to the reaction at the same time. This increases the number of reactions. For example, in the case of five bacterial species, 25 different reactions are required, combining each of the five test samples with each of the five reference samples. Adding target microorganisms after the primer set has been constructed requires significant effort and cost.

In the droplet digital PCR (ddPCR) method, the number of target microorganisms is limited to, for example, four. Pre-processing steps, including the creation of droplets, are complicated and time-consuming. Primers must be designed so that a non-specified PCR amplification product does not occur. Adding target microorganisms after the primer set has been constructed requires significant effort and cost. Measuring devices have a high price and running cost.

In the MF qPCR method, the number of target microorganisms is limited to, for example, 12. Measuring devices have very high prices and running costs.

As described above, the methods of the comparative examples have various problems. In view of the problems described above, the measurement method according to the present disclosure is described below.

In the measurement method according to an embodiment of the present disclosure, primers required for nucleic acid amplification are easily designed. Many varieties of microorganisms are quantitatively measured simultaneously. More than 12 different target microorganisms are quantitatively measured simultaneously. The cost of the method for simultaneous quantitative measurement of multiple target microorganisms is reduced. Even when the number of target microorganisms increases, the cost of testing does not increase. There is no need to perform aseptic operation or other operations requiring expertise. In other words, a testing technique with excellent operability is provided. Measurement results may be output in sufficiently short time that feedback may be provided in time for bioprocess or production process control. Microorganisms that cannot be cultured or that have lost their ability to grow due to damage may be quantitatively measured. The identification of target microorganisms and quantitative measurement of the target microorganisms are performed simultaneously. Addition of target microorganisms may be easily implemented.

### (Example of measurement procedures)

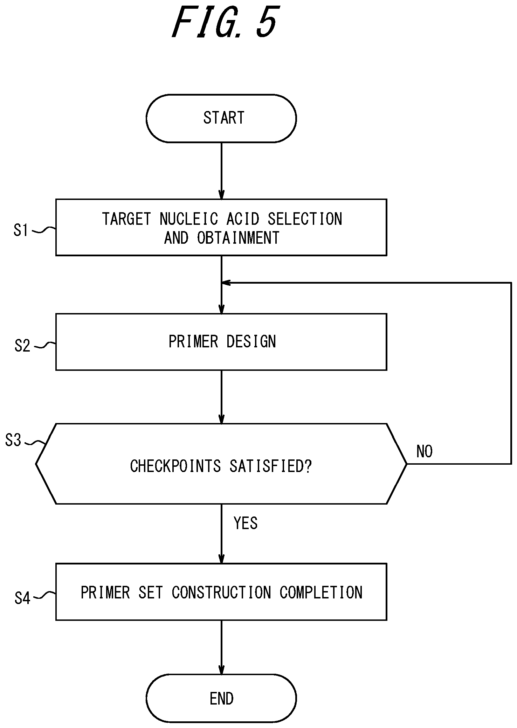

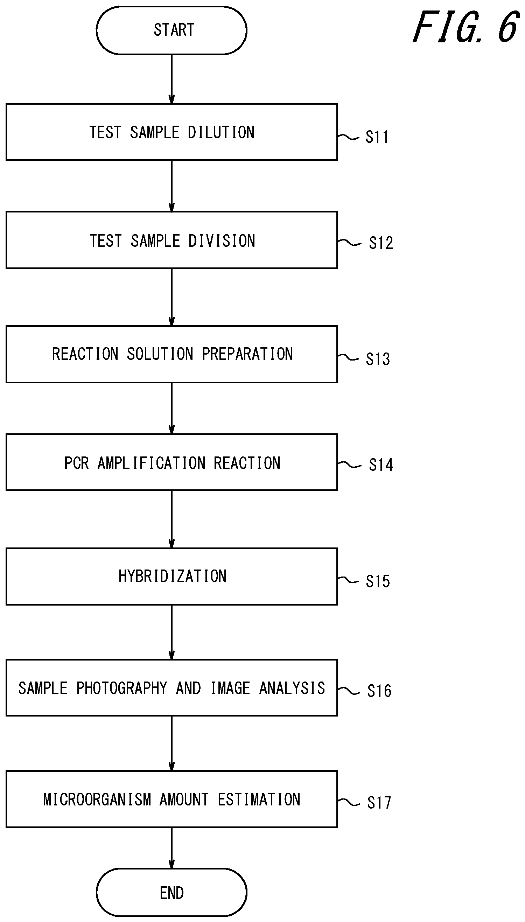

The measurement method according to an embodiment of the present disclosure is performed according to the procedures of the flowcharts illustrated in FIG. 5 and FIG. 6. First, pre-processing for quantitative measurement of microorganisms present in the test sample is described with reference to FIG. 5.

In the pre-processing, nucleic acid sequences for identifying and measuring target microorganisms are selected and obtained (step S1). Specifically, nucleic acid species that may be used to identify each microorganism in all targeted microorganism varieties are selected. Genomic deoxyribonucleic acid (DNA) is generally used as the target nucleic acid species. However, the nucleic acid species to be targeted is not limited to genes, and may be anything that may be used to identify the targeted microorganism, such as types of ribonucleic acid (RNA), mitochondrial DNA, or the like. The nucleic acid species may be selected from publicly available information or from nucleic acid sequence information obtained independently from existing nucleic acid sequencing methods for the target microorganism. The nucleic acid sequence may be obtained based on a nucleic acid sequence database or independently sequenced nucleic acid sequence information of the target nucleic acid.

Next, primers for amplifying the target nucleic acid are designed based on the nucleic acid sequence selected and obtained in the procedure of step S1 (step S2). Specifically, primers for PCR amplification reactions are designed based on target nucleic acid sequences to identify each target microorganism. Primers may be designed to have PCR amplified product lengths from 60 bp to 1 kbp. Further, the annealing temperature of each primer may be designed to be within ±5 °C.

Next, whether the primers designed in the procedure of step S2 satisfy the checkpoints to confirm that the target nucleic acid may be amplified is determined (step S3). Specifically, each PCR amplification reactions is performed to confirm that the target nucleic acid is efficiently amplifiable by the designed primers. The PCR amplification reaction is performed in a buffer solution containing the template nucleic acid, bases, and elongase required for the reaction, as well as salts required for the enzymatic reaction, all based on publicly available information. Further, the PCR amplification reaction is controlled by a temperature cycle appropriate for the enzyme used, consisting of three steps: annealing, extension, and thermal denaturation. The temperature cycle may consist of two steps where extension and thermal denaturation are performed at the same temperature. Following the PCR amplification reaction, the production of PCR amplification product is confirmed by gel electrophoresis or the like. Checkpoints include amount of amplification, amplification length, the presence or absence of amplification of non-specified products, the presence or absence of primer dimer formation, and the like. When checkpoints do not satisfy requirements, an improvement or redesign of the primers is performed. Whether the improved or redesigned primers are capable of amplifying the target nucleic acid is then checked.

When confirmation is obtained for individual primers, the primers designed to satisfy the checkpoint conditions are determined to be individual stand-alone primers. Further, when multiple varieties of nucleic acids are to be detected simultaneously, individual stand-alone primers are constructed for each variety of nucleic acid, and then whether checkpoint conditions are satisfied is checked for the primer set that is a mix of the individual stand-alone primers constructed for each variety of nucleic acid. The checkpoints for PCR amplification with the primer set may be the same as those for PCR amplification with individual stand-alone primers.

When PCR amplification with the primer set does not satisfy the checkpoint conditions, due to the production of a non-specified product for example, (step S3: NO), processing returns to step S2 and the individual stand-alone primer is redesigned or improved. Specifically, when amplification of the target nucleic acid is not confirmed, or when the amplification performance is extremely low, the checkpoint conditions are determined to be unsatisfied. When the checkpoint conditions are unsatisfied for an individual primer, the primer that does not satisfy the conditions is redesigned or improved. When the checkpoint conditions for each individual stand-alone primer are satisfied, confirmation is performed for the primer set that is a mix of each of the individual stand-alone primers. When the checkpoint conditions are unsatisfied for the primer set, the individual stand-alone primers are redesigned or improved in whole or in part.

When the checkpoint conditions are satisfied (step S2: YES), the construction of a primer set in which non-specified products do not occur is completed (step S4). The primer set may be constructed such that one primer corresponds to one target nucleic acid. The primer set may be constructed so that one common primer corresponds to multiple target nucleic acids.

Here, when the multiplex PCR described as a comparative example is performed, simultaneous multiple amplification reactions are necessarily performed on the primer set constructed in steps S1 through S4 to confirm that there is no production of primer dimers and non-specified products, or that such production does not affect subsequent processing.

In contrast, the use of a nucleic acid probe array to identify the amplified nucleic acids in the method according to the present embodiment eliminates the confirmation procedure required in multiplex PCR. Nucleic acid probe arrays, commonly referred to as DNA arrays, are measurement devices for nucleic acid detection in which target nucleic acid capture probes having complementary sequences to target nucleic acid probes are arranged on a solid phase so that each target nucleic acid can be identified. Artificially synthesized DNA having nucleic acid sequences complementary to naturally occurring DNA is used for the fixed nucleic acid probes. In addition to DNA, artificially produced glycol nucleic acid (GNA), locked nucleic acid (LNA), bridged nucleic acid (BNA), peptide nucleic acid (PNA), threose nucleic acid (TNA), and the like may be used as nucleic acid probes in the measurement method according to the present disclosure.

Next, the process of quantitatively measuring microorganisms present in the test sample is described with reference to FIG. 6.

First, the test sample to be measured is subjected to at least three steps of step dilution (step S11). Next, the step-diluted test sample is divided into at least three parts (step S12). Next, a reaction solution is prepared by adding to the divided samples the nucleic acid elongase required for the PCR amplification reaction, four bases, the primer set constructed in steps S1 through S4 of FIG. 5, and buffer (step S13). The buffer is added to maintain the pH of the reaction solution by increasing the activity of the nucleic acid elongase. Before the reaction solution is added in the procedure of step S13, template nucleic acids may be extracted by applying a nucleic acid extraction method from existing microorganisms. Further, nucleic acids may be extracted prior to the procedure in step S11. The divided sample or reaction solution is also referred to as a first sample.

Next, the reaction solution prepared in the procedure of step S13 is subjected to PCR amplification reaction (step S14). The reaction solution after the PCR amplification reaction has been performed is also referred to as a second sample. The PCR amplification reaction may be performed using a thermal cycler. The PCR amplification reaction may be performed as a typical PCR amplification reaction in which forward and reverse primers are added in a one-to-one ratio. Further, an asymmetric PCR amplification reaction may be applied for the purpose of increasing the efficiency of the hybridization reaction performed in the procedure of step S15 described below, or for the purpose of omitting the single-stranding process performed before the hybridization reaction. Asymmetric PCR reaction is a technique that changes the ratio of forward primer to reverse primer.

In the description above, PCR reaction has been used as an example of a nucleic acid amplification technique. However, nucleic acid amplification reactions are not limited to PCR. Known nucleic acid amplification techniques may be used, such as nucleic acid amplification testing (NAAT), transcription-reverse transcription concerted reaction (TRC), loop-mediated isothermal amplification (LAMP), rolling circle amplification (RCA), strand displacement amplification (SDA), nucleic acid sequence-based amplification (NASBA), ligase chain reaction (LCR), transcription mediated amplification (TMA), Smart Amplification Process (SmartAmp), isothermal and chimeric primer-initiated amplification of nucleic acids (ICAN), and the like.

Next, a reaction is performed between the nucleic acids amplified in the procedure of step S14 and the nucleic acid probe array (step S15). The nucleic acid probe array is a device in which probes having complementary sequences to target nucleic acids specific to the microorganism to be measured are fixed on a solid phase surface. The solid phase surface on which the nucleic acid probes are fixed is typically configured as, but not limited to, a flat substrate. It suffices that the solid phase surface is solid and capable of fixing each probe for separation and identification, and examples may include particles, thread strings, and the like. The flat substrate is typically made of glass, but is not limited to this, and may be made of metal, synthetic resin, paper-like material such as filter paper, and the like. When nucleic acids are amplified by PCR, the amplified product is obtained in a double-stranded state bound to the complementary strand. The nucleic acid probe array has multiple spots. To each spot, a probe with a complementary sequence to the target nucleic acid of a different variety of microorganism may be fixed. Each spot is configured to hold the nucleic acid probe on the solid phase surface without detaching. Further, spots are configured to be spatially separated from each other so that different varieties of probes fixed to spots are distinguishable from each other.

In the procedure of step S15, double strand reformation (hybridization) is performed with the nucleic acid probe having a sequence complementary to one strand of the double strand of the amplified product. Target nucleic acids are measured as reformation (hybridization) is performed. Therefore, a single-stranding treatment may be performed as a pretreatment in the procedure of step S15 for the purpose of increasing the reaction efficiency in the procedure of step S15. Treatment by heating or adjustment of pH may be applied as a single-stranding treatment.

Next, the sample for which reformation (hybridization) was performed in the procedure of step S15 is photographed, and the photographed image is analyzed (step S16). According to the image analysis, the number of samples containing nucleic acids that reacted with the nucleic acid probes in the hybridization process are counted.

Detection of typical nucleic acid probe arrays is performed by fluorometric measurement. In fluorometric measurement, primers for the PCR amplification reaction added in the procedure of step S13 are typically fluorescently labeled. In this method, a fluorescent dye may be attached as a label, but the method is not limited to this. A small molecule such as biotin may be attached to the primers as a label. In this case, labeling of a detection labeling agent may be detected by an additional procedure of reacting with the detection labeling agent containing a fluorescent label or the like before the imaging procedure in step S16. Further, the use of labels as described above increases measurement costs or operating procedures. Therefore, a nucleic acid probe array able to detect target nucleic acids without labeling may be used.

Next, the amount of microorganisms contained in the test sample is estimated by referencing the number of samples counted in step S16, the dilution rate of the step dilution performed in step S11, and the number of samples divided in step S12 in the most probable number table (step S17). By performing steps S16 and S17, the variety of microorganism may be identified based on light emission of the spot to which the probe with which hybridization was performed is fixed, and the amount of microorganism present may be quantitatively measured by the most probable number method.

As explained above, the amount of microorganisms contained in the test sample is measured by performing the measurement method according to the present embodiment. According to the measurement method of the present embodiment, the amounts of multiple target microorganisms are measured simultaneously. In the measurement method according to the present embodiment, target nucleic acids are amplified. The measurement method is based on a nucleic acid probe array. The nucleic acid probe array makes measuring target nucleic acids possible without being affected by the formation of non-specified products in the nucleic acid amplification process. As a result, the labor required for primer design in the pre-processing of nucleic acid amplification may be significantly reduced. Further, the measurement method is based on a nucleic acid probe array, and therefore measuring many varieties of nucleic acids simultaneously is possible. As a result, measuring the amounts of multiple varieties of microorganisms in a single reaction at the same time is possible. Further, the ability to measure the amounts of multiple varieties of microorganisms in a single reaction may greatly reduce the cost of measurement.

According to the comparative examples, the cost of measurement increases as the number of target microorganisms increases. However, in the measurement method according to the present embodiment, the cost of measurement does not increase even when the number of target microorganisms increases. Similarly, when there is a need to add a target microorganism after constructing a primer set for measurement, the target microorganism may easily be added to the same reaction system.

In the measurement method according to the present embodiment, the target microorganisms may be measured without culturing them. As a result, microorganisms for which culture methods have not been established may be used for measurement without requiring pre-culturing or other pretreatment. Further, damaged bacteria and the like that have been damaged by environmental stress, heat treatment, sterilization with chemicals, or other sterilization or disinfection treatments, making it difficult for the target microorganisms to grow even under optimal conditions for growth, may be used for measurement without requiring pretreatment. Further, the measurement method according to the present embodiment being culture-independent eliminates the time required for culturing. As a result, measurement results are obtainable in 30 minutes to several hours, for example.

The target microorganisms may be any microorganisms that contains nucleic acids, such as bacteria, fungi or archaea, or viruses, protozoa, or microscopic insects. Further, microorganisms in stages that do not immediately begin to multiply, such as bacteria spores, fungi spores, protozoa cysts, insect eggs, and the like may be measured.

According to the measurement method of the present embodiment, measurement results are obtained in a short time, which enables feedback to continuously operating processes such as production processes, treatment processes, and the like. Specifically, for example, the presence of contaminating bacteria other than those used for fermentation in a microbial fermentation process is known to reduce fermentation efficiency. Fermentation is a relatively fast reaction process. Therefore, when a test for contaminating bacteria based on a culture method is performed according to any of the comparative examples, the test results for the presence of contaminating bacteria are not obtained before the fermentation process is completed. By executing the measurement method according to the present embodiment, the presence of contaminating bacteria may be confirmed before the fermentation process is completed. When contaminating bacteria is present, countermeasures may be rapidly implemented, such as the addition of a chemical effective in controlling the contaminating bacteria. As a result, the decrease in fermentation efficiency may be suppressed.

### <Review>

The advantages of the measurement method according to the present embodiment are summarized below.

### < <Measurement method based on nucleic acid probe array> >

In the measurement method according to the present embodiment, the nucleic acid probe array is applied as a detection method, and therefore many varieties of microorganisms are targetable simultaneously. Measuring from one to several hundred targets simultaneously is possible. In a measurement method based on a typical PCR assay, when multiple target varieties are to be measured simultaneously, the design of primers used in the pre-processing of nucleic acid amplification must be strictly designed to suppress the formation of dimers or non-specified products. Therefore, the number of varieties that are measurable simultaneously is practically limited to a few varieties. According to the measurement method of the present embodiment, the measurement method is based on a nucleic acid probe array, thereby eliminating the effects of dimer and non-specified amplification product production. Accordingly, confirming that the target nucleic acids of individual target microorganisms are amplified is sufficient for the primer design in the process of simultaneously amplifying a variety of nucleic acids. As a result, the time and effort required to design primers may be significantly reduced.

Further, in the methods of the comparative examples, the cost of testing increases in proportion to the increase in the number of target microorganisms. In the measurement method according to the present embodiment, primers for target microorganisms and nucleic acid probes for detection are required for each target microorganism. However, these are used in miniscule amounts. As a result, the cost of testing does not increase significantly for an increase in the number of target microorganisms.

Further, when a method according to a comparative example is a measurement method based on a culture or PCR reaction, limiting the target microorganisms before measurement is necessary. In such cases, unexpected or unknown (unidentified) microorganisms cannot be measured. In the measurement method according to the present embodiment, being able to measure a large number of microorganisms simultaneously means a wide range of target microorganisms are targetable in advance. As a result, the odds of overlooking detection of microorganisms may be reduced. Further, in designing nucleic acid probes for detection, designing probes with a broad testing spectrum is possible. For example, by designing probes for regions commonly present between different bacteria, measurement targets are not only limited to specific bacteria, and comprehensive targeting of bacteria is possible. This makes detection of even unexpected or unknown bacteria possible.

### <<Dealing with many varieties simultaneously>>

In the measurement method according to the present embodiment, the multiplex PCR method may be applied to simultaneously measure multiple varieties of target microorganisms.

### < <Culturing not required> >

In the measurement method according to the present embodiment, culturing is not required, which allows testing to be performed in a short time, for example, from 30 minutes to within a few hours. The ability to make measurements in a short time makes it possible to feed back measurement results to production or treatment processes in bioprocesses and the like. Microorganisms for which culture methods have not been established, or VBNCs, may also be targeted for measurement. Further, the need to limit target microorganisms prior to measurement is eliminated. As a result, a wide range of microorganisms may be measured. Further, measurement may be performed by workers who do not have specialized skills such as aseptic operation.

### < <Method based on nucleic acid amplification>>

In the measurement method according to the present embodiment, a nucleic acid amplification method such as a PCR method may be applied to amplify the target nucleic acid and then perform measurement. As a result, measurement sensitivity is high.

### < <Applications> >

According to the measurement method of the present embodiment, microorganisms present in the test sample are quantitatively measured by nucleic acid measurement based on the most probable number method. The measurement targets nucleic acids, and therefore the target of the measurement is not limited to microorganisms. Measurement of anything containing nucleic acid is possible. For example, application to quantitative evaluation of genes expressed in certain cells, quantitative measurement of nucleic acid biomarkers in liquid biopsy, testing for food adulteration, and checking for contamination of products of genetic modification are possible. Food adulteration checks include, for example, checking meat marketed as beef for the meat of other species, or checking particular rice varieties for other varieties or rice considered unsuitable for sale. Adulteration checks include inspection of the ratio of adulteration. Inspections for food adulteration also include inspections for halal certification. The measurement method according to the present embodiment is not limited to the above examples and may be employed in any field where there is demand for quantitative measurement of target nucleic acids.

The measurement method according to an embodiment may further include a step of simultaneously quantitatively measuring the presence and amount of more than 12 varieties of microorganisms. In this way, the number of measurement cycles is reduced. As a result, convenience in microorganism measurement is improved.

The measurement method according to an embodiment may further include a step of designing a primer to be added to the first sample based on a variety of probe fixed in the probe array. In this way, target microorganisms may be easily changed. As a result, convenience in microorganism measurement is improved.

### (Example configuration of measurement system 1)

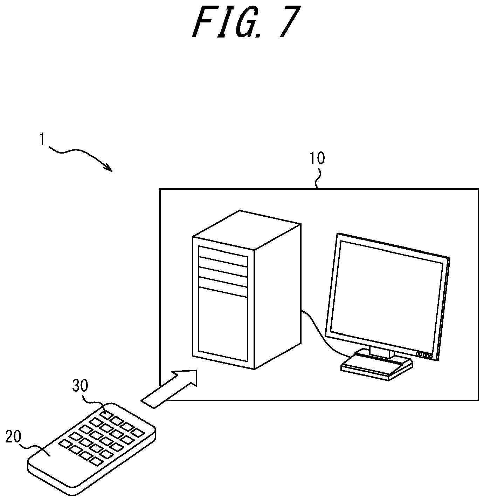

The measurement method according to the present embodiment may be implemented in various forms. Of the measurement methods, the procedures illustrated in FIG. 6 of photographing the sample and analyzing the image in step S16 and estimating amounts of microorganisms in step S17 may be implemented by a measurement system 1 illustrated in FIG. 7, FIG. 8, and FIG. 9.

As illustrated in FIG. 7, the measurement system 1 according to an embodiment includes a measurement device 10 and a probe array 20. The probe array 20 has a plurality of spots 30.

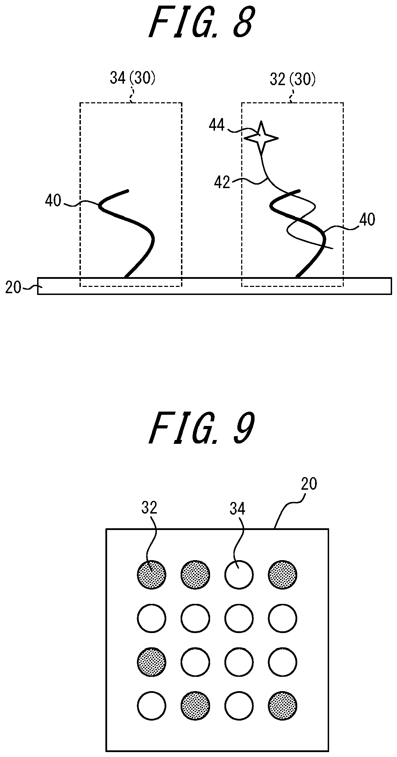

As illustrated in FIG. 8, the probe array 20 is a device in which probes 40 having complementary sequences to target nucleic acids 42 to be measured are fixed on a solid phase surface. The probes 40 are fixed to the spots 30 in one-to-one correspondence. The probes 40 may be fixed so that different varieties of the target nucleic acids 42 are hybridized at each of the spots 30. The probes 40 may be fixed so that the same variety of the target nucleic acids 42 is hybridized at a plurality of the spots 30.

According to the present embodiment, the target nucleic acids 42 having fluorescent labels 44 are hybridized with the probes 40. When one of the probes 40 is hybridized to one of the target nucleic acids 42 with one of the fluorescent labels 44, light emission is detected from the one of the spots 30 to which the one of the probes 40 is fixed. The spots 30 where light emission is detected are also referred to as light-emitting spots 32. When one of the probes 40 is not hybridized with the target nucleic acids 42, no light emission is detected from the spot 30 to which the probe 40 is fixed. The spots 30 where no light emission is detected are also referred to as non-light-emitting spots 34.

By excitation of the light-emitting spots 32 in the probe array 20 and detection of fluorescence, the light-emitting spots 32 and the non-light-emitting spots 34 may be discriminated, as illustrated in FIG. 9. Discrimination of the spots 30 may be performed by inserting the probe array 20 into the measurement device 10, as illustrated in FIG. 7.

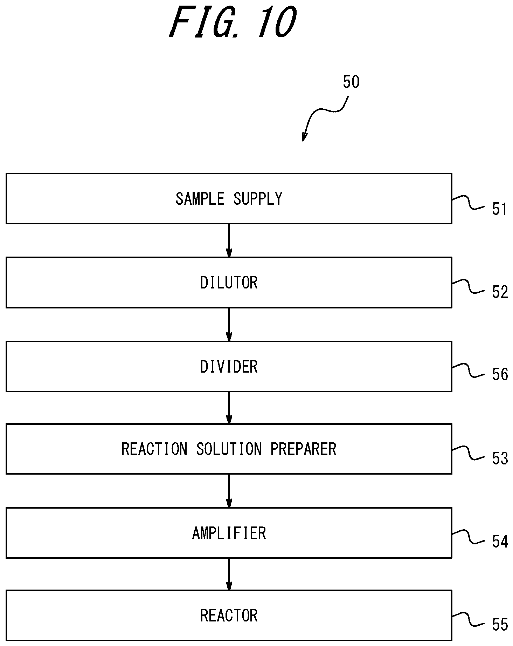

The measurement system 1 may be further configured to perform the process of preparing the probe array 20 in which the target nucleic acids 42 are hybridized to the probe 40 as a pretreatment step of the measurement method performed by the measurement system 1 illustrated in FIG. 7, FIG. 8, and FIG. 9. Specifically, the measurement system 1 may further include a pretreatment device 50 having a sample supply 51, a dilutor 52, a reaction solution preparer 53, an amplifier 54, and a reactor 55, as illustrated in FIG. 10.

The sample supply 51 is connected to the dilutor 52 via a flow path. The sample supply 51 supplies the test sample to the dilutor 52. The dilutor 52 stores a dilution solution in advance, and dilutes the test sample by internally mixing the test sample supplied from the sample supply 51 with the dilution solution. In other words, the procedure of step S11 of the flowchart in FIG. 6 may be performed by the dilutor 52. A diluted test sample is also referred to as a diluted sample.

The dilutor 52 is connected to the reaction solution preparer 53 via a flow path. The dilutor 52 supplies the diluted sample to the reaction solution preparer 53. The reaction solution preparer 53 stores the reaction solution in advance, and prepares the reaction solution by internally mixing the diluted sample supplied from the dilutor 52 with the reaction solution. In other words, the procedure of step S13 of the flowchart in FIG. 6 may be performed by the reaction solution preparer 53.

The pretreatment device 50 may further include a divider 56 that divides the diluted sample on the flow path connecting the dilutor 52 and the reaction solution preparer 53. In other words, the procedure for dividing the diluted sample, indicated as step S12 in the flowchart in FIG. 6, may be performed by the divider 56. The procedure for dividing the diluted sample may be performed by the reaction solution preparer 53.

The amplifier 54 provides PCR amplification of the reaction solution prepared in the reaction solution preparer 53. In other words, the procedure of step S14 of the flowchart in FIG. 6 may be performed by the amplifier 54.

The reactor 55 provides reaction of the PCR amplified reaction solution from the amplifier 54 with the probe array 20. In other words, the procedure of step S15 of the flowchart in FIG. 6 may be performed by the reactor 55. The reactor 55 may have a mechanism to take in and eject the probe array 20. The reactor 55 may have a flow path that allows the PCR amplified reaction solution to flow into an area where the probe array 20 is installed.

The pre-processing of preparing the probe array 20 in which the target nucleic acids 42 are hybridized with the probes 40 may be performed by the pretreatment device 50 described above. The measurement system 1 may measure microorganisms in test samples based on the probe array 20 prepared by the pretreatment device 50.

The pretreatment device 50 may control the temperature of each component. The pretreatment device 50 may further include a heater, for example.

The flow paths connecting the components of the pretreatment device 50 may be composed of silicone or other deformable materials. In this way, a configuration that pumps liquid by narrowing or widening the flow paths may be achieved.

Each component of the pretreatment device 50 may be configured as a microfluidic device.

The reactor 55 may have a mechanism for draining the reaction solution to remove unreacted reaction solution from the spots 30 of the probe array 20 after the hybridization reaction. The probe array 20 itself may have a mechanism for draining the reaction solution. The reactor 55 may have a mechanism to replace the probe array 20 with another probe array 20.

The present disclosure is not limited to the configurations specified in the embodiments described above, and various modifications are possible without departing from the scope of the claims. For example, the functions and the like included in each step may be reconfigured as long as no logical inconsistency arises. Multiple steps may be combined into one, and a single step may be split into more than one.

### REFERENCE SIGNS LIST

- 1: Measurement system
- 40: Measurement device
- 20: Probe array
- 30: Spots (32: light-emitting spots, 34: non-light-emitting spots)
- 40: Probes
- 42: Target nucleic acids
- 44: Fluorescent labels
- 50: Pretreatment device (51: sample supply, 52: dilutor, 53: reaction solution preparer, 54: amplifier, 55: reactor, 56: divider)

## Claims

1. A measurement method for quantitatively measuring amounts of at least two varieties of microorganisms present in a test sample, the measurement method comprising:
a step of producing a first sample by performing step dilution and division of the test sample;
a step of producing a second sample by amplifying nucleic acids of the microorganisms in the first sample;
a step of performing hybridization of the second sample in a probe array including spots to which probes having complementary sequences to target nucleic acids specific to each of the varieties of the microorganisms are fixed; and
a step of identifying the varieties of the microorganisms based on light emission from the spots where the hybridization is performed and quantitatively measuring amounts of the microorganisms by a most probable number method.

2. The measurement method of claim 1, further comprising a step of designing a primer to be added to the first sample based on a variety of probe fixed in the probe array.

3. A measurement system for quantitatively measuring amounts of at least two varieties of microorganisms present in a test sample, the measurement system comprising:
a pretreatment device and a measurement device, wherein
the pretreatment device is operable to:
produce a first sample by performing step dilution and division of the test sample;
produce a second sample by amplifying nucleic acids of the microorganisms in the first sample;
perform hybridization of the second sample in a probe array including spots to which probes having complementary sequences to target nucleic acids specific to each of the varieties of the microorganisms are fixed; and
identify the varieties of the microorganisms based on light emission from the spots where the hybridization is performed and quantitatively measure amounts of the microorganisms by a most probable number method.
